(19) Europäisches Patentamt / European Patent Office / Office européen des brevets

(11) **EP 3 970 597 B1**

(12) **EUROPEAN PATENT SPECIFICATION**

(45) Date of publication and mention
of the grant of the patent:
**31.12.2025   Bulletin 2026/01**

(21) Application number: **20197466.4**

(22) Date of filing: **22.09.2020**

(51) International Patent Classification (IPC):
*A61B 1/00* *(2006.01)*      *A61B 1/05* *(2006.01)*
*A61M 16/04* *(2006.01)*     *A61B 1/015* *(2006.01)*
*A61B 1/018* *(2006.01)*

(52) Cooperative Patent Classification (CPC):
**A61B 1/05; A61B 1/00082; A61B 1/00096;**
**A61M 16/0404; A61M 16/0434; A61M 16/0459;**
**A61M 16/0477; A61M 16/0486; A61M 16/0488;**
A61B 1/00048; A61B 1/015; A61B 1/018;
A61M 2205/3569; A61M 2205/3592;
A61M 2205/502;                                   (Cont.)

(54) **VISION CATHETER**

SEHKATHETER

CATHÉTER DE VISION

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(43) Date of publication of application:
**23.03.2022   Bulletin 2022/12**

(73) Proprietor: **Ambu A/S**
**2750 Ballerup (DK)**

(72) Inventors:
• **SØRENSEN, Morten**
  **2750 Ballerup (DK)**
• **NIELSEN, Lars Ulrik**
  **2830 Virum (DK)**

(74) Representative: **COPA Copenhagen Patents**
**Rosenvængets Allé 25**
**2100 Copenhagen Ø (DK)**

(56) References cited:
EP-B1- 1 683 468          JP-A- S63 136 781
KR-A- 20180 138 037     US-A1- 2017 280 988

(52) Cooperative Patent Classification (CPC): (Cont.)
A61M 2205/583; A61M 2205/587

**Description**

TECHNICAL FIELD

The present disclosure relates to a catheter comprising a vision device.

BACKGROUND

A catheter is a thin tube made from medical grade materials with one or more lumens allowing drainage, administration of fluids or gases, access by surgical instruments and performs a wide variety of other tasks depending on the type of catheter. The catheter is intended to be inserted in a body cavity, duct, or vessel, whereupon it is typically desired to verify that the catheter is correctly positioned inside the body. A way of allowing such verification is to include a small vision device enabling an operator to view images captured by the vision device thereby verifying that the catheter is in the desired location. However, in such a prior art vision catheter a relatively large portion of the field of view of the vision device is occupied by the catheter which does not provide much information to the operator. In particular, some catheters may comprise an inflatable cuff positioned distally relative to the camera which may obstruct a large portion of the field of view of the vision device.

SUMMARY

On this background, it may be seen as an object of the present disclosure to provide a low-cost catheter with a vision device with an improved field of view.

Document US 2017/280988 shows a vision catheter for arthroscopy, wherein the window closing the lumen of the catheter is not orthogonal to the lumen axis. In an alternative embodiment, a device for inspection and treatment of the uterine cavity is disclosed, the device having an instrument lumen and a vision lumen.

One or more of these objects may be met by aspects of the present disclosure as described in the following.

A first aspect of this disclosure relates to a catheter having a distal end for insertion into a patient, and comprising:

- a catheter tube enclosing at least a first catheter lumen and a vision lumen separated from the first catheter lumen, the first catheter lumen having a first distal opening at the distal end of the catheter;
- a window closing off a distal end of the vision lumen and having an interior window surface facing the vision lumen and an exterior window surface; and
- a vision device positioned in the vision lumen and comprising a vision module, such as a camera with an image sensor or an optical fibre, having an optical axis extending through the window;

wherein the interior window surface and/or the exterior window surface is/are non-orthogonal with respect to the optical axis of the vision module, and wherein the interior window surface and the exterior window surface are non-parallel.

By arranging at least one of the window surfaces angled with respect to the optical axis and both window surfaces non-parallel, a view direction of the vision module may thus be, upon passing the window, effectively refracted away from the catheter so as to show more of the desired anatomy and less of the static catheter tube thus providing an effective view direction. The effective view direction extends outwardly away from the catheter and is angled with respect to the optical axis of the vision module and thereby also a central line of the catheter tube. An advantage of such an arrangement is that the catheter tube may be manufactured by an extrusion process, which is typically cheap, and further a lens arrangement of the vision device for this purpose can be omitted and thus a standard vision device can be used.

In this disclosure, the angle or orthogonality between an axis, e.g. the optical axis of the vision module, and a surface is performed by measuring or calculating the smallest angle between the optical axis and a normal direction of the surface, i.e. a surface normal, preferably in a plane defined by a central line of the first catheter lumen and the optical axis.

Additionally or alternatively, the optical axis and/or a central line of the vision lumen may be parallel with respect to a central line of the catheter tube, e.g. a central line of the first catheter lumen, adjacent to the window.

Additionally or alternatively, the window may be positioned proximally with respect to the distal opening of the first catheter lumen.

Additionally or alternatively, the window or the window surfaces may be adapted to not focus or disperse light transmitted therethrough. This may for example be achieved by the window surfaces being planar and thus having no lens effect.

Additionally or alternatively, the vision lumen may be closed off at a distal end by the window. The window may form an integral part of the catheter tube. Alternatively, the window may form part of the vision device.

Additionally or alternatively, the vision device may be arranged so that the vision module is viewing through the window. Alternatively, the vision device may be arranged so that a line of view of the vision module is extending through the window.

Additionally or alternatively, the window may comprise a window material, such as polycarbonate, having a refraction index different from air. The window material may additionally be coated on the interior and/or the exterior surface. The refraction index of polycarbonate may be 1.58.

Additionally or alternatively, a refractive index of window material, e.g. polycarbonate, may be higher than a refractive index of an internal medium, e.g. air, between the window and vision module, and wherein an angle of a surface normal of the interior window surface with respect to a ray incident on the interior window surface from the effective view direction as refracted by the exterior window surface may be less than a critical angle.

A critical optical angle $\Theta_c$ may be defined as the smallest angle of incidence that yields total reflection. This can happen when light propagates from an interior medium of a refraction index to an exterior medium of a lower refraction index, such as when light propagate from the window material (which is typically polycarbonate with a refraction index of 1.58) to the medium external of the catheter (which is typically air with a refraction index of 1). The critical angle $\Theta_c$ for isotropic media may be found using the following formula:

$$\Theta_c = \sin^{-1}\left(\frac{n_2}{n_1}\right)$$

Wherein $n_1$ is the refraction index of the internal medium, $n_2$ is the refraction index of the exterior medium, and $n_2 \le n_1$

Additionally or alternatively, the window may be adapted to refract an effective view direction of the vision module away from the catheter tube. This may e.g. be achieved by angling the interior window surface away from the central line of the catheter and/or by angling the exterior window surface towards the central line of the catheter.

Additionally or alternatively, the interior and/or exterior window surface may be substantially planar. Additionally or alternatively, the interior window surface and the exterior window surface may be substantially planar and non-parallel. In the context of this disclosure, substantially planar may be understood as a surface being optically planar so that light propagating through said surface is not focused nor dispersed but refracted.

Additionally or alternatively, the exterior window surface may be non-orthogonal with respect to the optical axis and optionally the interior window surface may be orthogonal with respect to the optical axis.

Additionally or alternatively, an angle $\beta_{ext}$ between a surface normal of the exterior window surface and the optical axis may be in the range 1° to 45°, 2° to 40°, 3° to 35°, 4° to 30°, 5° to 20°, or 5° to 10°.

Additionally or alternatively, the interior window surface may be non-orthogonal with respect to the optical axis and optionally the exterior window surface may be orthogonal with respect to the optical axis.

Additionally or alternatively, an angle $\beta_{int}$ between a surface normal of the interior window surface and the optical axis may be in the range 1° to 45°, 2° to 40°, 3° to 35°, 4° to 30°, 5° to 20°, or 5° to 10°.

Additionally or alternatively, the vision device may be arranged with an air gap separating the vision device and/or vision module from the interior window surface. The vision device may be arranged to view through the air gap. This may provide the advantage that redirecting the effective view direction of the vision module is more consistent as refraction occurs between predetermined media, i.e. air and the window material.

Additionally or alternatively, the vision lumen may extend substantially in parallel to the first catheter lumen and/or a second catheter lumen.

Additionally or alternatively, the vision device may be arranged so that the optical axis is parallel to a longitudinal axis of the catheter. The catheter lumen(s) and/or the vision lumen may extend in parallel to the longitudinal axis of the catheter.

Additionally or alternatively, the interior window surface and the exterior window surface may be adapted so that an effective view direction of the vision module is angled/refracted with respect to the optical axis by an angle, $\alpha$, in the range 1° to 15°, preferably in the range 2° to 12°, more preferably in the range 3° to 10°, or even more preferably in the range of 5° to 8°. A redirection of the view direction in these ranges has been found to provide an increasingly optimised trade-off between decreasing the view occupied by the catheter tube while retaining enough to allowing navigating the catheter to the desired location inside the body.

Such an angle, $\alpha$, of the effective view direction with respect to the optical axis may be obtained by choosing suitable angles for the window surfaces with respect to the optical axis and a window material for the window with a suitable refraction index. When the window surfaces are planar, the angle of the effective view direction for given angles of respective surface normals of the window surfaces with respect to the optical axis may be calculated using Snell's Law.

For instance, when the interior window surface is orthogonal with respect to the optical axis (i.e. $\beta_{int} = 0$), the angle, $\alpha$, of the effective view direction with respect to the optical axis for a given angle $\beta_{ext}$ of the surface normal of the exterior window surface with respect to the optical axis may be calculated using Snell's law:

$$\alpha = \sin^{-1}\left(\frac{n_{ext}}{n_{win}}\sin\beta_{ext}\right) - \beta_{ext}$$

Wherein $n_{ext}$ is the refraction index of the medium exterior of the catheter, typically air with a refraction index of 1, $n_{win}$ is the refraction index of the window material, which is typically polycarbonate having a refraction index of 1.58, and $\beta_{ext}$ is the angle of the surface normal of the exterior window surface with respect to the optical axis.

The skilled person will appreciate the angle of the effective view direction can be derived from Snell's law for other embodiments. Such as when both window surfaces are non-orthogonal with respect to the optical axis and when the exterior window surface is orthogonal with respect to the optical axis while the angle $\beta_{int}$ of the surface normal of the interior window surface with respect to the optical axis is non-zero.

Additionally or alternatively, the catheter tube may comprise a flush inlet connected via one or more flush channels to respective flush openings oriented so that pressurised flush fluid supplied to the flush inlet flows through the flush channel(s) and exits through the flush openings directed towards the exterior surface of the window.

The vision device comprises a housing which includes the window and forms a sealed compartment encompassing the vision module. The housing and the window may be integrally formed optionally as a single housing element. The housing may be retained in the vision lumen by a friction fit and/or adhesively. Such a housing may have the advantage of providing a single component which may be tested prior to assembly with the remaining parts of the catheter.

Additionally or alternatively, the catheter tube may comprise a plastic material adapted to be bendable by an operator. The plastic material may be selected from the group consisting of: polyvinyl chloride (PVC) optionally with a plasticizer to ensure desired flexibility, e.g. when bending the catheter tube; thermoplastic elastomer (TPE) such as styrene ethylene butylene styrene (SEBS) or styrene butylene styrene (SBS); thermoplastic polyurethane (TPU); thermoplastic vulcanizate (TPV); rubber such as natural rubber, synthetic rubber, butyl rubber, nitrile rubber, latex, neoprene, or isoprene; silicone; or mixtures thereof. Most thermoplastic vulcanizates (TPVs) are binary blends of polyolefins and thermoplastic diene elastomers. Other elastomers sometimes used in TPVs include butyl rubber, natural rubber or nitrile rubber blended with isotactic polypropylene (iPP).

Additionally or alternatively, the catheter tube may be extruded.

Additionally or alternatively, the catheter may further comprise a first inflatable cuff optionally positioned proximally relative to the window. The catheter may be a single-lumen endotracheal tube or double-lumen endotracheal tube.

Additionally or alternatively, the catheter may comprise a second catheter lumen having a second distal opening, wherein the distal opening of the second lumen is positioned distally with respect to the distal opening of the first catheter lumen. Such a catheter may also be known as a double-lumen endotracheal tube. The second catheter lumen may be separate from the first catheter lumen and the vision lumen.

Additionally or alternatively, the catheter may comprise a second inflatable cuff positioned between the distal opening of the second catheter lumen and the window. The catheter may comprise the first inflatable cuff. The catheter may be a double-lumen endotracheal tube.

Additionally or alternatively, the catheter may be an endotracheal tube, such as a double-lumen endotracheal tube or a single-lumen endotracheal tube. Single- or double-lumen refers in this case to the number of catheter lumens of the endotracheal tube which are the lumens used for administration of substances, ventilation or other medical procedures. Such endotracheal tubes may comprise one or more additional lumen for other purposes, e.g. as in the present case, a vision lumen, lumen(s) for inflating/deflating the inflatable cuff(s), and/or optionally one or more flushing lumen for flushing the window.

Additionally or alternatively, the vision device comprises a cable for transmitting image signals extending from the vision module and through at least part of the vision lumen. Alternatively, the vision device may comprise a wireless transmitter for wirelessly communicating to a monitor.

Additionally or alternatively, a catheter vision system may comprise a catheter according the first aspect of this disclosure and a monitor, wherein the catheter is connectable to the monitor via one or more cables or via a wireless connection, e.g. a standard radiofrequency wireless connections such as Bluetooth, Wi-Fi, etc. the monitor includes a display unit adapted to display an image captured by the vision device.

A person skilled in the art will appreciate that any one or more of the above aspects of this disclosure and embodiments thereof may be combined with any one or more of the other aspects of this disclosure and embodiments thereof.

BRIEF DESCRIPTION OF THE DRAWINGS

Embodiments of this disclosure will be described in more detail in the following with regard to the accompanying figures. The figures show one way of implementing the present invention and are not to be construed as being limiting to other possible embodiments falling within the scope of the attached claim set.

Fig. 1 is a schematic perspective illustration of a catheter in the form of a single-lumen endotracheal tube,
Fig. 2 is a schematic perspective illustration of the distal end of the catheter of Fig. 1,
Fig. 3 is a schematic perspective illustration of a catheter in the form of a double-lumen endotracheal tube,
Fig. 4 is a schematic perspective illustration of the distal end of the catheter of Fig. 3,
Fig. 5 is a schematic cross-sectional illustration of a distal end of the catheter of Fig. 3,
Fig. 6 is a schematic perspective illustration of a vision device for implementation in the catheter of Figs. 1-2 or in the catheter of Figs. 3-5,
Fig. 7 is a schematic exploded illustration of the vision device of Figs. 6-7,
Fig. 8 is a schematic cross-sectional illustration of a first embodiment of the vision device of Figs. 6-7,
Fig. 9 is a schematic cross-sectional illustration of a distal end of the first embodiment of the vision device shown in Fig. 8,
Fig. 10 is a schematic cross-sectional illustration of a second embodiment of the vision device of Figs. 6-7,
Fig. 11 is a schematic detail illustration of a distal end of the second embodiment of the vision device shown in Fig. 10, and
Fig. 12 is a schematic illustration of a monitor.

## DETAILED DESCRIPTION OF THE INVENTION

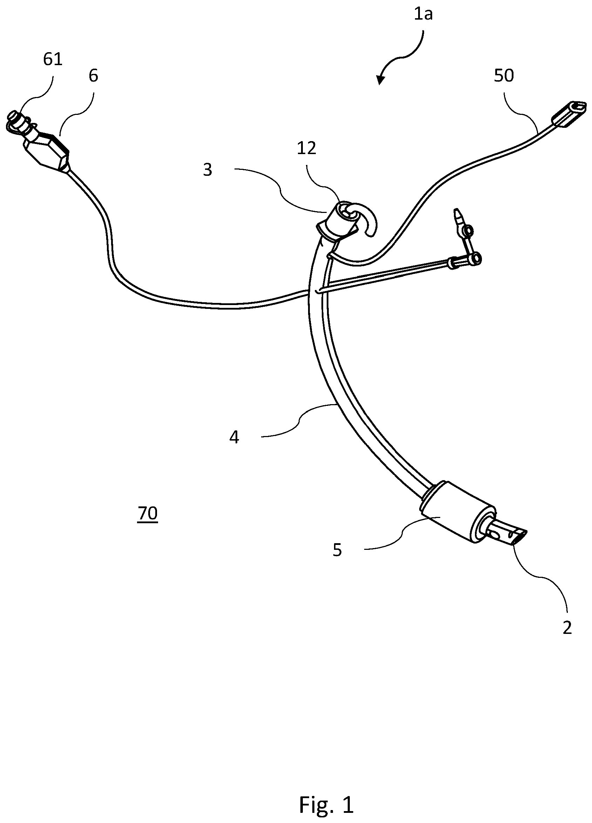

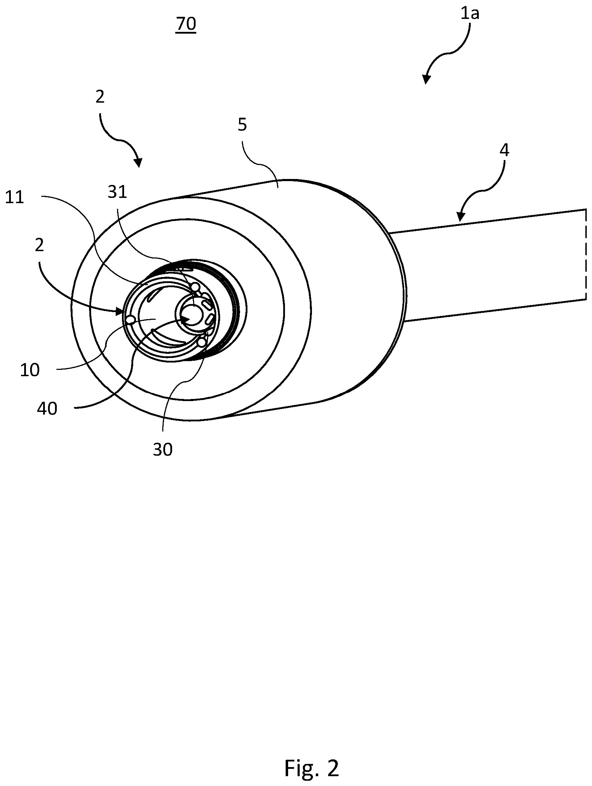

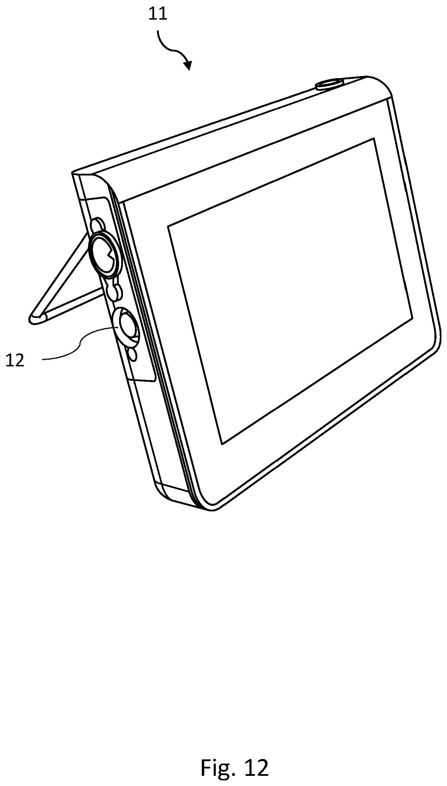

Figs. 1 and 2 illustrate a first catheter 1a in the form of a single-lumen endotracheal tube. As can best be seen in Fig. 1, the catheter 1a comprises an extruded catheter tube 4 extending from a proximal end 3 to a distal end 2. The catheter 1a further comprises a first cuff 5 inflatable via an inflation tube, which at an end comprises a first valve 61 and a first pilot balloon 6. The first valve 61 comprises an inlet for connection to a pressure source such as a syringe and the first pilot balloon 6 provides a tactile indication of the pressure inside the first cuff 5. As best seen in Fig. 2, the catheter tube 4 encloses a first catheter lumen 10 having a first distal opening 11 at the distal end 2 of the catheter tube 4 and a first proximal opening 12 at the proximal end 3 of the catheter tube 4 as seen in Fig. 1. The catheter tube 4 further comprises a vision lumen 30 extending in parallel to and separate from the first catheter lumen 10. A vision device 40 is positioned in the vision lumen 30 and comprises a camera having an image sensor and a lens stack, one or more light sources 48, and a window 31. The window 31 closes off a distal end of the vision lumen 30 from an external medium 70, typically air. The distal end of the vision lumen 30 is located adjacent to the first distal opening 11 of the first catheter lumen 10 as seen in Fig. 2. The vision device 40 comprises a plug 50 (as seen in fig 1.) connectable to a socket 52 of monitor 51, which is shown in Fig. 12, for displaying images captured by the vision device 40 on the monitor 51. Alternatively, the vision device 40 could be wirelessly connected to the monitor 51.

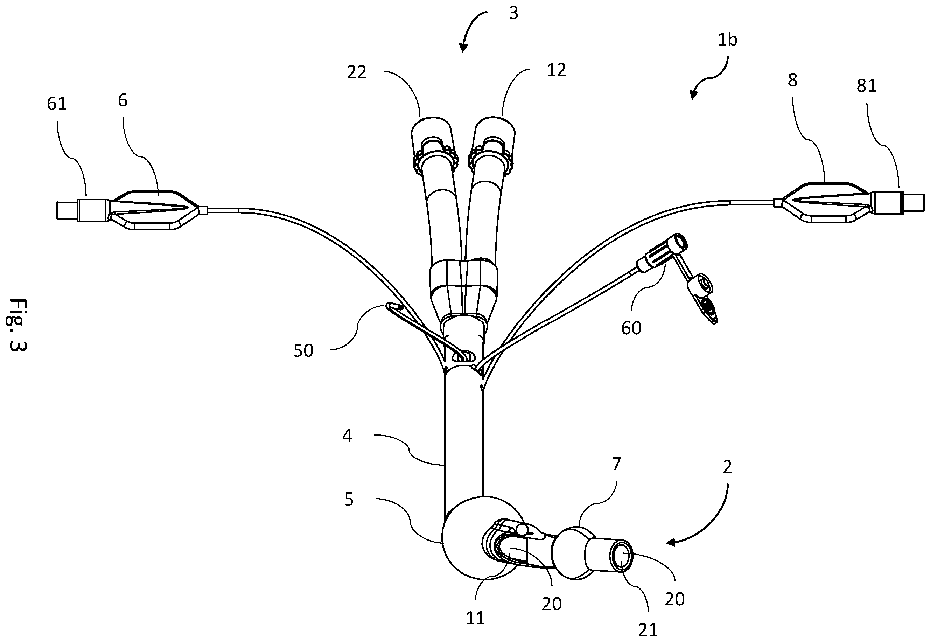

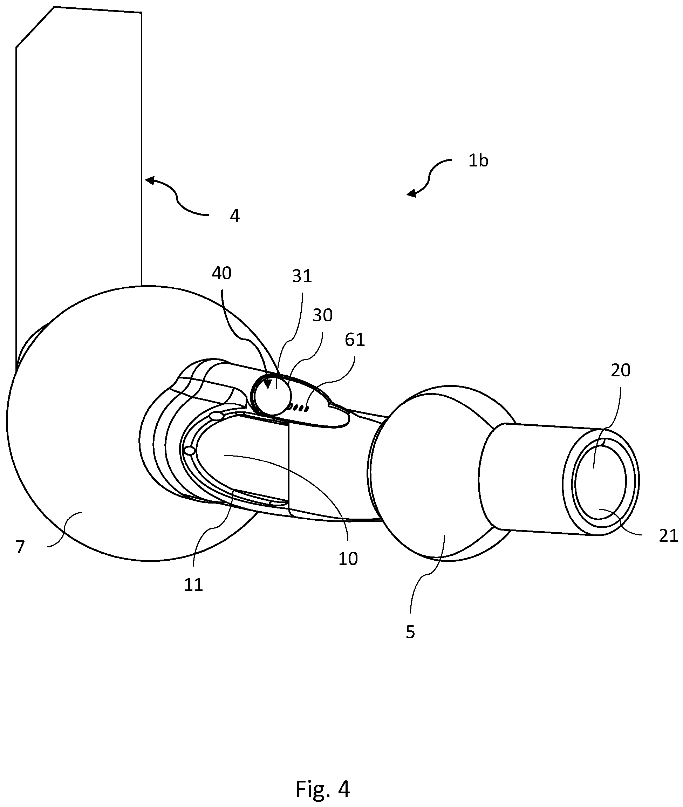

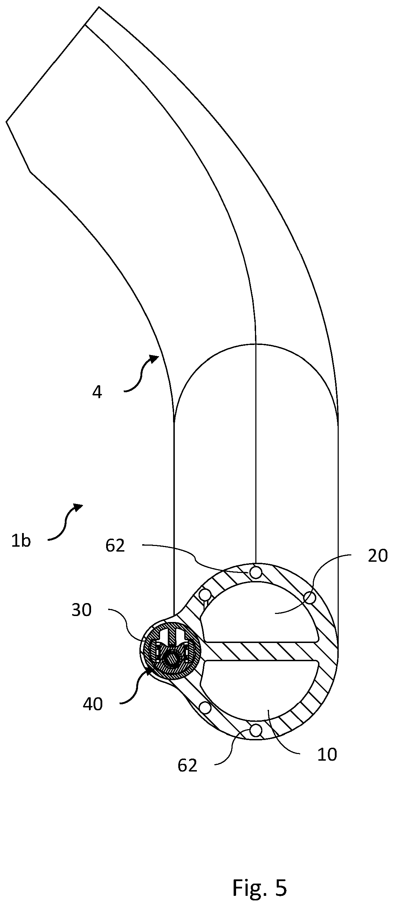

Figs. 3-5 illustrates a second catheter 1b in the form of a double-lumen endotracheal tube. The second catheter 1b comprises an extruded catheter tube 4 similar to the catheter tube 4 of the first catheter 1a as shown in Figs. 1 and 2 but differs therefrom by further comprising a second catheter lumen 20 extending between a second proximal opening 22 at the proximal end 3 of the catheter tube 4 and a second distal opening 21 at the distal end 2 of the catheter tube 4. The second catheter lumen 20 is adjacent to and separate from the first catheter lumen 10 and the vision lumen 30 as best seen in Fig 5. As seen in Fig. 4 and similar to the first catheter 1a, the vision device 40 is located at a distal end of the dedicated vision lumen 30 adjacent to the first distal opening 11 of the first catheter lumen 10. The vision device 40 closes off the distal end of the vision lumen 30 and is tightly retained in the vision lumen 30 by a friction fit. The catheter 1 further comprises a second cuff 7 inflatable via an inflation tube, which at an end comprises a second valve 81 and a second pilot balloon 8. The second cuff 7 is positioned between the first distal opening 11 and the second distal opening 21. The second valve 81 comprises an inlet for connection to a pressure source such as a syringe and the second pilot balloon 8 provides a tactile indication of the pressure inside the second cuff 7. Furthermore, by means of a flush inlet 60, the window 31 of the vision device 40 may be flushed via flush openings 61 formed in the catheter tube 2, as best seen in Fig. 4. The flush inlet 60 is connected to the flush openings 61 via a number of dedicated flush channels 62 co-extruded in an outer wall of the catheter tube 4 as illustrated in Fig. 5. As in the first catheter 1a, the vision device 40 comprises a plug 50 connectable to a socket 52 of monitor 51, which is shown in Fig. 12, for displaying images captured by the vision device 40 on the monitor 51. Alternatively, the vision device 40 could be wirelessly connected to the monitor 51.

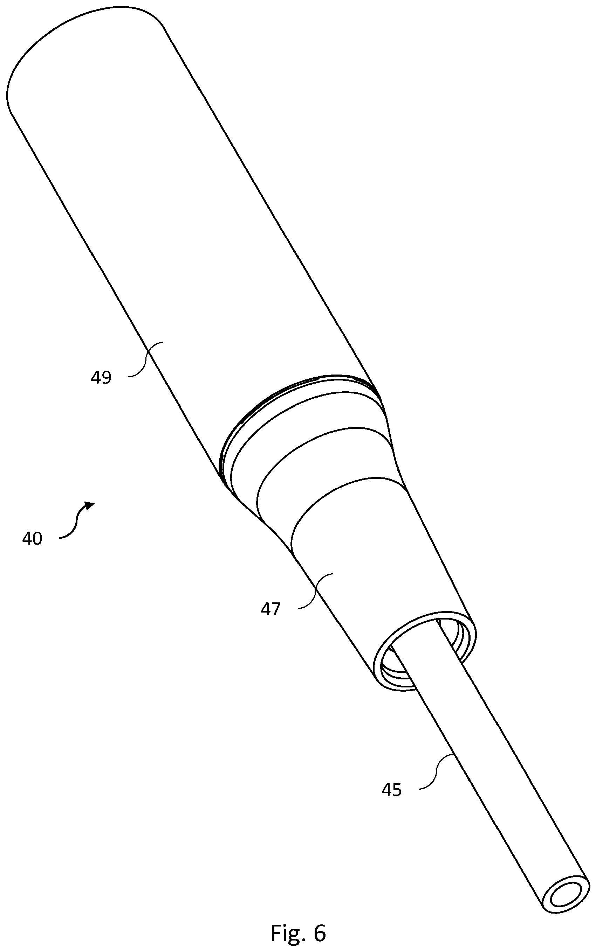

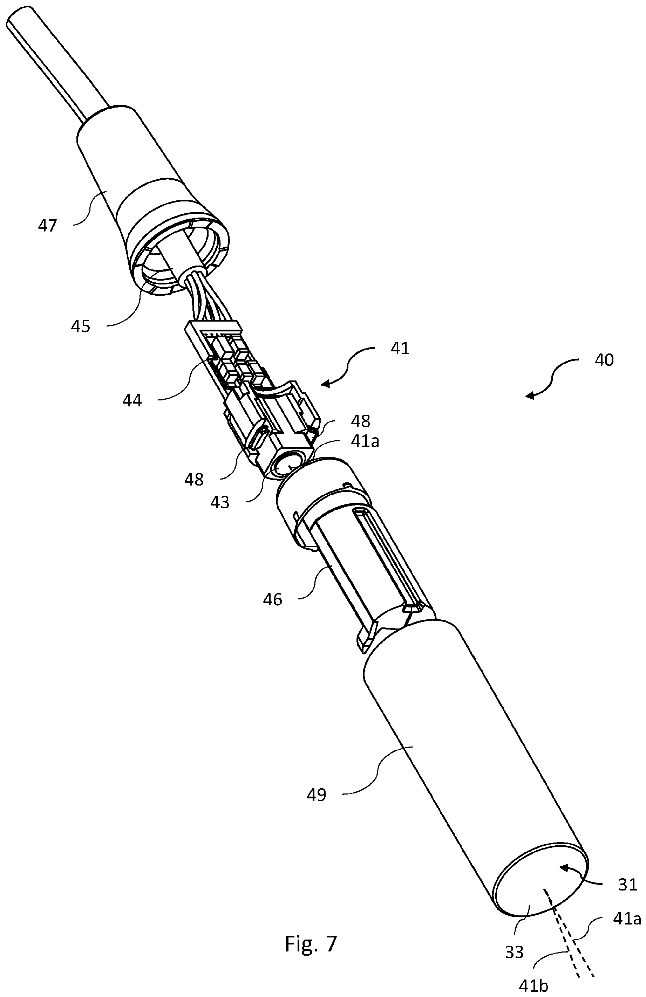

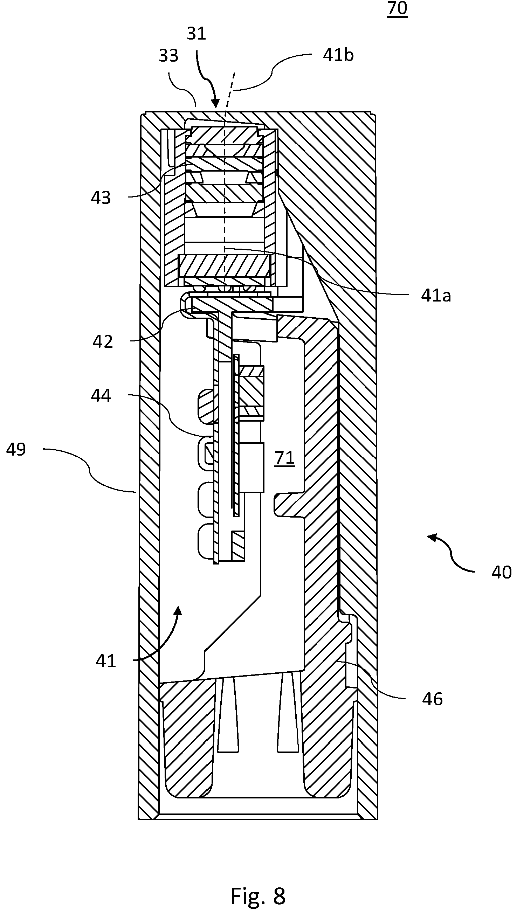

The vision device 40 is illustrated in Figs. 6-8 and 10 and comprises a housing including a housing wall 49 and an end cover 47. The housing wall 49 and end cover 47 are adhered together to form a sealed compartment which encompasses a vision module 41 held by a holder 46. The housing wall 49 has a window 31 integrally moulded so the housing wall 49 and window 31 forms a single element. The window 31 comprises a planar exterior window surface 33 and a planar interior window surface 31, which is shown in Figs. 8-11. The vision module 41 comprises a lens stack 43, e.g. of three lenses, two light sources 48, and an image sensor 42 with an optical axis 41a extending through the lens stack 43 and the window 31. The light sources 48 is supplied with power by the printed circuit board 44 and provide illumination for the image sensor 42. The image sensor 42 is electrically connected to a printed circuit board 44 for processing image signals from the image sensor 42 and transmitting these signals, e.g. through a cable 45, to a monitor 51 as shown in Fig. 12 for displaying the images to the operator. Alternatively, image signals could be transmitted unprocessed from the image sensor 42 to an external image processing unit (not shown), which could be positioned in the handle or in the monitor.

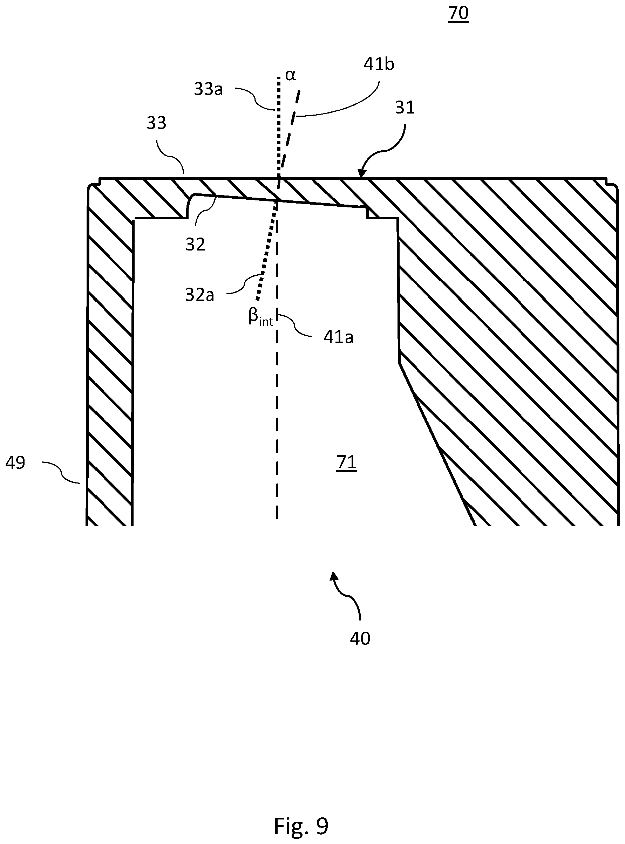

A first embodiment of the window 31 is schematically illustrated in Figs. 8-9. As best seen in Fig. 9, the exterior window surface 33 of the window 31 is planar and has a surface normal 33a parallel to the optical axis 41a of the image sensor 42 of the vision module 41. A surface normal 32a of the interior window surface 32 forms an angle, $\beta_{int}$, of about 8.5° with respect to the optical axis 41a of the vision device 40.

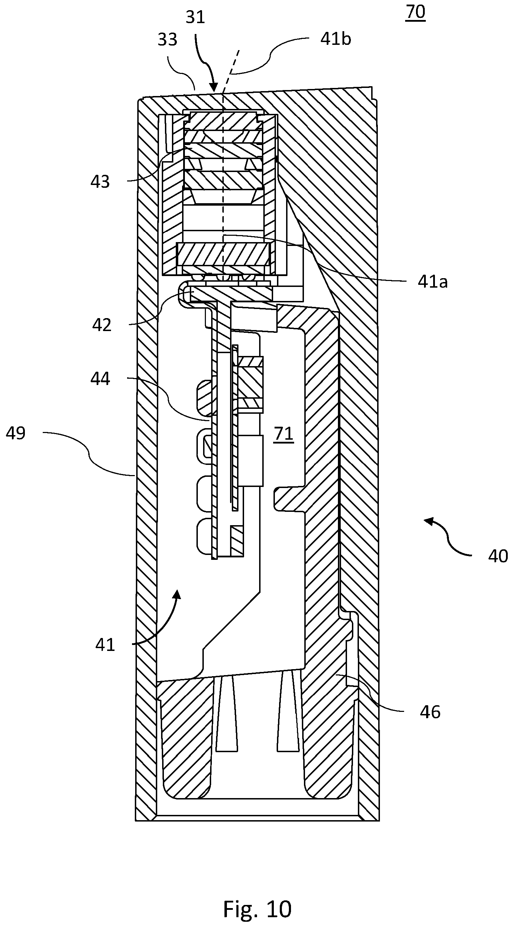

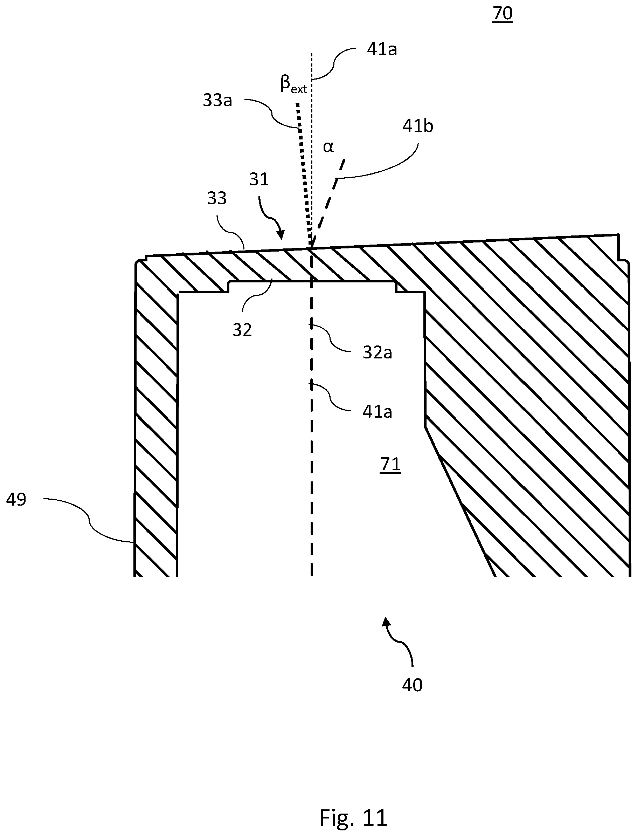

A second embodiment of the window 31 is schematically illustrated in Figs. 10-11. As best seen in Fig. 11, the interior window surface 32 of the window 31 has a surface normal 32a parallel to the optical axis 41a. The exterior window surface 33 has a surface normal 33a forming an angle, $\beta_{ext}$, of about 8.5° with respect to the optical axis 41a.

A third embodiment of the window is not illustrated but corresponds to a combination of the first and second embodiment. In the third embodiment, the interior window surface 32 and the exterior window surface 33 are planar and non-parallel. Further, the surface normal 32a of the interior window surface 32 forms an angle, $\beta_{int}$, of up to about 4° - 5° with respect to the optical axis 41a of the vision device 40, while the surface normal 33a of the exterior window surface 33 forms an angle, $\beta_{ext}$, of up to about 4° - 5° with respect to the optical axis 41a.

In all three embodiments, an external medium 70 of air surrounds the catheter, the vision lumen 30 encloses an internal medium 71 of air, and the window 31 consists essentially of polycarbonate with a refraction index of 1.58. The window 33 refracts the effective view direction 41b away from the catheter tube 4 by an angle $\alpha$ of about 5° with respect to the optical axis 41a. This leads to a larger fraction of the image captured by the vision device 40 to be occupied by the inspected anatomy. Each of the above described embodiments can be implemented in the first catheter 1a shown in Figs. 1-2 or second catheter 1b shown in Figs. 3-5. For illustrative purposes, the angles between surface normals, the optical axis, and the effective view angles are schematically exaggerated and may be smaller in practice.

An angle $\alpha$ between the optical axis 41a and the effective view direction 41b of about 5° - 8° has been found to provide an adequate trade-off between decreasing the view occupied by the catheter tube 4 while retaining enough to allowing navigating the catheter to the desired location. The skilled person will appreciate that this angle is achieved by choosing the combination of the window refraction index and angles between the respective surface normals 32a, 33a and the optical axis 41a.

LIST OF REFERENCES

| | |
|---|---|
| 1a | first catheter |
| 1b | second catheter |
| 2 | distal end |
| 3 | proximal end |
| 4 | catheter tube |
| 5 | first inflatable cuff |
| 6 | first pilot balloon |
| 61 | first valve |
| 7 | second inflatable cuff |
| 8 | second pilot balloon |
| 81 | second valve |
| 10 | first catheter lumen |
| 11 | first distal opening |
| 12 | first proximal opening |
| 20 | second catheter lumen |
| 21 | second distal opening |
| 22 | second proximal opening |
| 30 | vision lumen |
| 31 | window |
| 32 | interior window surface |
| 32a | surface normal |
| 33 | exterior window surface |
| 33a | surface normal |
| 40 | vision device |
| 41 | vision module |
| 41a | optical axis |
| 41b | effective view direction |
| 42 | image sensor |
| 43 | lens stack |
| 44 | printed circuit board |
| 45 | cable |
| 46 | holder |
| 47 | end cover |
| 48 | light source |
| 49 | housing wall |
| 50 | plug |
| 51 | monitor |
| 52 | socket |
| 60 | flush connection |
| 61 | flush opening |
| 62 | flush channel |
| 70 | external medium |
| 71 | internal medium |
| $\alpha$ | angle of effective view direction with respect to the optical axis |
| $\beta_{int}$ | angle of the surface normal of the interior window surface with respect to the optical axis |
| $\beta_{ext}$ | angle of the surface normal of the exterior window surface with respect to the optical axis |

**Claims**

1. A catheter having a distal end for insertion into a patient, and comprising:

    a catheter tube (4) enclosing at least a first catheter lumen (10) and a vision lumen (30) separated
    from the first catheter lumen, the first catheter lumen having a first distal opening at the distal end of the catheter;
    a housing including a window (31) closing off a distal end of the vision lumen and having an interior window surface (32) facing the vision lumen and an exterior window surface (33), the housing being retained in the vision lumen; and
    a vision device positioned in the vision lumen and comprising a vision module (41), such as a camera with an image sensor or an optical fibre, having an optical axis extending through the window, wherein the housing forms a sealed compartment encompassing the vision module;

    wherein the interior window surface and/or the exterior window surface is/are non-orthogonal with respect to the optical axis of the vision module, and wherein the interior window surface and the exterior window surface are non-parallel.

2. A catheter according to any one of the previous claims, wherein the window is adapted to refract an effective view direction of the vision module away from the catheter tube.

3. A catheter according to any one of the previous claims, wherein the interior and/or exterior window surface is/are substantially planar.

4. A catheter according to any one of the previous claims, wherein the exterior window surface is non-orthogonal with respect to the optical axis and optionally the interior window surface is orthogonal with respect to the optical axis.

5. A catheter according to any one of the previous claims, wherein an angle between a surface normal of the exterior window surface and the optical axis is in the range 1° to 45°.

6. A catheter according to any one of the previous claims, wherein the interior window surface is non-orthogonal with respect to the optical axis and optionally the exterior window surface is orthogonal with respect to the optical axis.

7. A catheter according to any one of the previous claims, wherein an angle between a surface normal of the interior window surface and the optical axis is in the range 1° to 45°.

8. A catheter according to any one of the previous claims, wherein the vision lumen extends substantially in parallel to the first catheter lumen.

9. A catheter according to any one of the previous claims, wherein the interior window surface and/or the exterior window surface are adapted so that an effective view direction of the vision module is angled with respect to the optical axis by an angle in the range 1° to 15°, preferably in the range 3° to 12°.

10. A catheter according to any one of the previous claims, wherein the catheter tube comprises a flush inlet connected via one or more flush channels to respective flush openings oriented so that pressurised flush fluid supplied to the flush inlet flows through the flush channel(s) exits through the flush openings directed towards the exterior surface of the window.

11. A catheter according to any one of the previous claims, wherein the housing and the window are integrally formed.

12. A catheter according to claim 11, wherein the housing of the vision device is retained in the vision lumen by a friction fit.

13. A catheter according to any one of the previous claims, wherein the catheter is an endotracheal tube, such as a double-lumen endotracheal tube or a single-lumen endotracheal tube.

14. A catheter vision system comprising a catheter according any one of the previous claims and a monitor, wherein the catheter is connectable to the monitor and the monitor is adapted to display an image captured by the vision device.

**Patentansprüche**

1. Katheter, aufweisend ein distales Ende zum Einführen in einen Patienten und umfassend:

einen Katheterschlauch (4), der mindestens ein erstes Katheterlumen (10) und ein Sichtlumen (30), das
vom ersten Katheterlumen getrennt ist, umschließt, wobei das erste Katheterlumen eine erste distale Öffnung am
distalen Ende des Katheters aufweist;
ein Gehäuse, das ein Fenster (31) einschließt, das ein distales Ende des Sichtlumens verschließt und
eine dem Sichtlumen zugewandte innere Fensterfläche (32) und eine äußere Fensterfläche (33) aufweist,
wobei das Gehäuse im Sichtlumen festgehalten wird; und
eine Sichtvorrichtung, die im Sichtlumen positioniert ist und ein Sichtmodul (41) umfasst, wie eine Kamera mit einem Bildsensor oder eine optische Faser, die eine optische Achse aufweist, die sich durch das Fenster erstreckt, wobei das Gehäuse einen abgedichteten Raum bildet, der das Sichtmodul umgibt;
wobei die innere Fensterfläche und/oder die äußere Fensterfläche in Bezug auf die optische Achse des Sichtmoduls nicht orthogonal ist/sind und wobei die innere Fensterfläche und die äußere Fensterfläche nicht parallel sind.

2. Katheter nach einem der vorstehenden Ansprüche, wobei das Fenster dazu angepasst ist, eine effektive Blickrichtung des Sichtmoduls vom Katheterschlauch weg abzulenken.

3. Katheter nach einem der vorstehenden Ansprüche, wobei die innere und/oder die äußere Fensterfläche im Wesentlichen eben ist/sind.

**4.** Katheter nach einem der vorstehenden Ansprüche, wobei die äußere Fensterfläche in Bezug auf die optische Achse nicht orthogonal ist und, optional, wobei die innere Fensterfläche in Bezug auf die optische Achse orthogonal ist.

**5.** Katheter nach einem der vorstehenden Ansprüche, wobei ein Winkel zwischen einer Oberflächennormalen der äußeren Fensterfläche und der optischen Achse im Bereich von 1° bis 45° liegt.

**6.** Katheter nach einem der vorstehenden Ansprüche, wobei die innere Fensterfläche in Bezug auf die optische Achse nicht orthogonal ist und, optional, wobei die äußere Fensterfläche in Bezug auf die optische Achse orthogonal ist.

**7.** Katheter nach einem der vorstehenden Ansprüche, wobei ein Winkel zwischen einer Oberflächennormalen der inneren Fensterfläche und der optischen Achse im Bereich von 1° bis 45° liegt.

**8.** Katheter nach einem der vorstehenden Ansprüche, wobei sich das Sichtlumen im Wesentlichen parallel zum ersten Katheterlumen erstreckt.

**9.** Katheter nach einem der vorstehenden Ansprüche, wobei die innere Fensterfläche und/oder die äußere Fensterfläche so angepasst sind, dass eine effektive Blickrichtung des Sichtmoduls in Bezug auf die optische Achse um einen Winkel im Bereich von 1° bis 15°, vorzugsweise im Bereich von 3° bis 12°, abgewinkelt ist.

**10.** Katheter nach einem der vorstehenden Ansprüche, wobei der Katheterschlauch einen Spüleinlass umfasst, der über einen oder mehrere Spülkanäle mit entsprechenden Spülöffnungen verbunden ist, die so ausgerichtet sind, dass unter Druck stehendes Spülfluid, das dem Spüleinlass zugeführt wird, durch den einen oder die mehreren Spülkanäle fließt und durch die Spülöffnungen, die zur äußeren Fläche des Fensters hin gerichtet sind, austritt.

**11.** Katheter nach einem der vorstehenden Ansprüche, wobei das Gehäuse und das Fenster einstückig gebildet sind.

**12.** Katheter nach Anspruch 11, wobei das Gehäuse der Sichtvorrichtung durch Reibungspassung im Sichtlumen festgehalten wird.

**13.** Katheter nach einem der vorstehenden Ansprüche, wobei der Katheter ein Endotrachealtubus ist, beispielsweise ein doppellumiger Endotrachealtubus oder ein einlumiger Endotrachealtubus.

**14.** Katheter-Sichtsystem, umfassend einen Katheter nach einem der vorstehenden Ansprüche und einen Monitor, wobei der Katheter mit dem Monitor verbindbar ist und der Monitor dazu angepasst ist, ein von der Sichtvorrichtung aufgenommenes Bild anzuzeigen.

**Revendications**

**1.** Cathéter présentant une extrémité distale pour insertion dans un patient, et comprenant :

un tube (4) de cathéter enfermant au moins une première lumière (10) de cathéter et une lumière de vision (30) séparée de la première lumière de cathéter, la première lumière de cathéter présentant une première ouverture distale à l'extrémité distale du cathéter ; un boîtier incluant une fenêtre (31) obturant une extrémité distale de la lumière de vision et présentant une surface de fenêtre intérieure (32) orientée vers la lumière de vision et une surface de fenêtre extérieure (33), le boîtier étant retenu dans la lumière de vision ; et un dispositif de vision positionné dans la lumière de vision et comprenant un module de vision (41), tel qu'une caméra avec un capteur d'image ou une fibre optique, présentant un axe optique s'étendant à travers la fenêtre, dans lequel le boîtier forme un compartiment étanche englobant le module de vision ; dans lequel la surface de fenêtre intérieure et/ou la surface de fenêtre extérieure est/sont non orthogonale(s) par rapport à l'axe optique du module de vision, et dans lequel la surface de fenêtre intérieure et la surface de fenêtre extérieure ne sont pas parallèles.

**2.** Cathéter selon l'une quelconque des revendications précédentes, dans lequel la fenêtre est adaptée pour réfracter une direction de vue effective du module de vision à l'écart du tube de cathéter.

**3.** Cathéter selon l'une quelconque des revendications précédentes, dans lequel les surfaces de fenêtre intérieure et/ou extérieure est/sont sensiblement plane(s).

**4.** Cathéter selon l'une quelconque des revendications précédentes, dans lequel la surface de fenêtre extérieure n'est pas orthogonale par rapport à l'axe optique et facultativement la surface de fenêtre intérieure est orthogonale par rapport à l'axe optique.

**5.** Cathéter selon l'une quelconque des revendications précédentes, dans lequel un angle entre une normale à la surface de la surface de fenêtre extérieure et l'axe optique est dans la plage de 1° à 45°.

**6.** Cathéter selon l'une quelconque des revendications précédentes, dans lequel la surface de fenêtre intérieure n'est pas orthogonale par rapport à l'axe optique et facultativement la surface de fenêtre extérieure est orthogonale par rapport à l'axe optique.

**7.** Cathéter selon l'une quelconque des revendications précédentes, dans lequel un angle entre une normale à la surface de la surface de fenêtre intérieure et l'axe optique est dans la plage de 1° à 45°.

**8.** Cathéter selon l'une quelconque des revendications précédentes, dans lequel la lumière de vision s'étend sensiblement en parallèle à la première lumière de cathéter.

**9.** Cathéter selon l'une quelconque des revendications précédentes, dans lequel la surface de fenêtre intérieure et/ou la surface de fenêtre extérieure sont adaptées de sorte qu'une direction de vue effective du module de vision soit inclinée par rapport à l'axe optique d'un angle dans la plage de 1° à 15°, de préférence dans la plage de 3° à 12°.

**10.** Cathéter selon l'une quelconque des revendications précédentes, dans lequel le tube de cathéter comprend une entrée de rinçage connectée via un ou plusieurs canaux de rinçage à des ouvertures de rinçage respectives orientées de sorte que le fluide de rinçage sous pression fourni à l'entrée de rinçage s'écoule à travers le ou les canaux de rinçage et sort par les ouvertures de rinçage dirigées vers la surface extérieure de la fenêtre.

**11.** Cathéter selon l'une quelconque des revendications précédentes, dans lequel le boîtier et la fenêtre sont formés d'une seule pièce.

**12.** Cathéter selon la revendication 11, dans lequel le boîtier du dispositif de vision est retenu dans la lumière de vision par un ajustement par friction.

**13.** Cathéter selon l'une quelconque des revendications précédentes, dans lequel le cathéter est une sonde endotrachéale, telle qu'une sonde endotrachéale à double lumière ou une sonde endotrachéale à simple lumière.

**14.** Système de vision de cathéter comprenant un cathéter selon l'une quelconque des revendications précédentes et un moniteur, dans lequel le cathéter peut être connecté au moniteur et le moniteur est adapté pour afficher une image capturée par le dis-

positif de vision.

Fig. 1

Fig. 2

Fig. 3

Fig. 4

Fig. 5

Fig. 6

Fig. 7

Fig. 8

Fig. 9

Fig. 10

Fig. 11

11

12

Fig. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017280988 A